# EUROPEAN PATENT SPECIFICATION

(11) **EP 0 789 555 B1**
(45) Date of publication and mention of the grant of the patent: **29.08.2001**
(21) Application number: 94910422.8
(22) Date of filing: 11.03.1994
(51) Int. Cl.: A61K 7/48, A61K 7/06, A61K 7/42, A61K 7/00, A61K 31/19

(54) **DIMERIC ACIDS IN COSMETIC OR PHARMACEUTICAL COMPOSITIONS**
DIMERE SÄUREN IN KOSMETIKA UND ARZNEIMITTELN
ACIDES DIMERES DANS DES COMPOSITIONS COSMETIQUES ET PHARMACEUTIQUES

(30) Priority: 12.03.1993 GB 9305163
(43) Date of publication of application: 20.08.1997
(73) Proprietor: The Boots Company PLC, Nottingham, Nottinghamshire NG2 3AA (GB)
(72) Inventor: BARTON, Stephen Peter, Nittingham NG2 3AA (GB)
(74) Representative: Thacker, Michael Anthony
(86) International application number: EP9400814
(87) International publication number: WO9420067

(56) References cited:
- EP-A- 0 584 692
- DE-A- 3 327 645
- GB-A- 1 439 244
- US-A- 3 846 550
- US-A- 3 981 990

## Description

The present invention relates to compositions which comprise a so-called "dimer acid". Such compositions may be emulsions that can be caused to thicken in use. The dimer acid also finds utility as an anti-free radical agent in pharmaceutical, cosmetics and toiletries compositions, such as sunscreens, moisturisers, skin and hair cleansers and conditioners, deodorants and antiperspirants.

DE-A-3327645 (Henkel) discloses the use of dimer acids in the preparation of liposome-like vesicles. The vesicles may comprise the cyclic dimeric acid mixture available under the trade name Versadyme. Versadyme is produced by a Diels Alder reaction between oleic acid and a diene thermally produced from linoic acid.

JP-A-58027636 (Shiseido) is reported as disclosing an oil-in-water type emulsion comprising dimer acid, stearic acid, cetanol, vaseline, squalene, glycerin monostearate, monosodium-N-stearoylglutamate, monosodium-N-myristoylglutoamate, propylene glycol, potassium hydroxide, sodium hydroxide and carboxyvinyl polymer.

US-3846550 (Akrongold) discloses cosmetic compositions comprising the dimer acid mixture available under the trade name Empol 1014. Empol 1014 is a mixture of 1 % w/w monomeric acid, 95% w/w dimeric acid and 4% w/w of other polymeric acids.

US-3981990 (Kelly) discloses skin protecting compositions comprising the cyclic dimeric acid mixture available under the trade name Empol 1022. Empol 1022 is a mixture of 3% w/w monomeric acid, 75% w/w dimeric acid and 22% w/w of other polymeric acids.

GB-1439244 (Procter & Gamble) discloses compositions comprising the dimeric acid mixture available under the trade name Empol 1018. Empol 1018 is a mixture of 83% w/w dimeric acid and 17% w/w of other polymeric acids.

EP-0584692 (Murer & Wurtz) [copending with the present application and published after the priority date of the present invention] discloses cosmetic compositions comprising the dimeric acid mixture available under the trade name Pripol 1009. Pripol 1009 is a mixture comprising dimeric acid and other polymeric acids.

None of the above prior art documents disclose cosmetic compositions comprising a linear dimeric acid substantially free of other polymeric acids, or disclose that such a linear dimeric acid may be an anti-free radical agent and form emulsions with the advantageous rheological properties described herein.

According to one aspect of the present invention there is provided cosmetic, pharmaceutical or toiletries compositions comprising a linear dicarboxylic acid (commonly known as a 'dimer acid') of the formula (I) 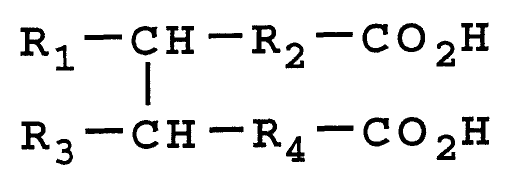 or an acceptable salt thereof;
wherein R₁ and R₃ are each independently C₆ to C₁₀ alkyl and R₂ and R₄ are each independently C₆ to C₁₀ alkylene; with the proviso that the acid comprises one or more unsaturated carbon-carbon bonds and is substantially free of other polymeric acids. Preferably the composition is in the form of an emulsion comprising an emulsifying agent chosen such that the emulsion may be shear-thickened in use.

Preferably the emulsifying agent is an alkoxylated emulsifier, suitably an alkoxylated C₁₄ to C₂₂ alkanol, preferably an ethoxylated alkanol, preferably a polyethoxylated alkanol.

Preferably R₁ has the same chain length as R₃, and R₂ has the same chain length as R₄. Suitably, R₁, R₂, R₃ and R₄ all have the same chain length, preferably being C₈ alkyl or alkylene.

Suitably, the dicarboxylic acid is producable by dimerisation of an unsaturated C₁₈ fatty acid, preferably linoleic acid. It will be appreciated, however, that other methods of synthesis are possible. Particularly preferred dicarboxylic acids are those available under the trade name 'Pripure' from Unichema International. Previously these compounds have been used in the materials and petroleum industries as lubricants and rust-proofing agents. They have also been used in the preparation of liposomes.

Suitably, the dicarboxylic acid is the acid available under the trade name 'Pripure 3780' from Unichema International.

Preferably the emulsifying agent is a mixture of alkoxylated C₁₄ to C₂₂ alkanols having different hydrophilic/lipophilic balances (HLBs).

Preferably, the emulsifying agent is a mixture of a polyethoxylated C₁₄ to C₂₂ alkanol bearing a chain of 2 to 10 ethoxy groups (HLB between about 4 and about 12) and a polyethoxylated C₁₄ to C₂₂ alkanol bearing a chain of 10 to 40 ethoxy groups (HLB between about 10 and about 20). Suitably these alkanols are present in a ratio of about 2 to 1 respectively. Suitably, in each case, the alkanol is a C₁₈ alkanol.

A preferred emulsifying agent is a mixture of POE (2) stearyl alcohol and POE (21) stearyl alcohol, suitably in a ratio of 2:1.

Suitably, the emulsifying agent is present in an amount of from 0.5% to 20%, suitably 2% to 10%, preferably about 3% by weight of the emulsion.

The dicarboxylic acid may be present in an amount of from 0.2% to 10%, preferably about 1% to 2.5% by weight of the emulsion. A corresponding dicarboxylic acid ester may also be present.

Suitably, the emulsifying agent and the dicarboxylic acid are present in a weight ratio of 5:1 to 1:1, preferably about 3:1.

Preferably, the emulsion is an oil-in-water emulsion. The emulsion is thin, stable and may show liquid crystalline phases.

Preferably, the emulsion further comprises a sphingolipid such as ceramide, sphingomyelins cerebrosides or gangliosides, of plant or animal origin, suitably in an amount of from 0.05% to 1%, suitably about 0.1% by weight.

Suitably, the emulsion also comprises a steroid such as the cholesterols, lanosterols, phytosterols, mycosterols and ergosterols, for example in an amount of from 0.05% to 1%, suitably about 0.15% by weight. Also useful as active components of the emulsion system are adrenocorticoids, oestrogens, androgens and bile acids.

The emulsion compositions provided by the present invention is of unusually low viscosity. They are stable and may show varying proportions of liquid crystalline phases. The rheological behaviour of the emulsion compositions when applied to the skin or hair is also unusual. Whilst initially watery, the emulsion composition rapidly thickens when subjected to shearing ('shear-thickening') and dries, penetrating the skin or hair surface and providing a high resistance to further massage. Once the product has been rubbed into the skin it imparts a smooth, non-tacky finish. Application of water, however, brings a soapy cleansing effect. Once dried the skin reverts to a smooth condition.

Preferred toiletries compositions of the present invention are those for application to the skin and hair, for example sunscreens, skin cleansers, moisturisers and cosmetics products. The rheological properties provided by the emulsion of the present invention are particularly valuable in these types of compositions.

The toiletries compositions of the present invention may be formulated, for example, as creams and lotions or as sprays, such as aerosols and pump sprays. The emulsion compositions of the present invention may, if desired, be made thin enough to be dispensed as a spray and subsequently thickened by agitation, especially shearing.

It will be understood that numerous further components can be added to the toiletries compositions of the present invention, for example, organic or inorganic sunscreen agents, such as microfine titanium dioxide, oils such as evening primrose oil and waxes such as mixed sphingoceryl wax.

In one embodiment of the invention, the emulsion is dispensed directly to the skin and shear thickened in place on the skin. Alternatively, the emulsion maybe shear-thickened before application to the skin.

Preferably, the composition is in the form of an emulsion and is dispensed in the form of a spray, suitably by use of aerosolising means. Thus, the invention also provides aerosolising means, such as an aerosol spray can or pump-spray, comprising a composition as defined above. The present composition, when in sprayable form, differs markedly from other sprayable emulsion compositions. Other emulsion compositions are normally thick and must undergo shear thinning in order to pass through a narrow orifice such as a spray nozzle. By contrast, the emulsion compositions of the present invention, when in sprayable form, may be made initally thin enough to pass through such a nozzle unchanged and may if desired undergo thickening only when subjected to shear on the skin or hair, for example by rubbing.

The emulsion compositions may also be dispensed by means of a "roller" mechanism, of the type commonly used for anti-perspirants and deodorants. Passage of the emulsion composition through the "roller" may generate a shearing action which may thicken the composition prior to application to the skin. Thus the invention also provides a "roller" dispenser comprising a composition as defined above.

The emulsion compositions of the present invention may be used in a method of cleansing and/or moisturising the skin by applying the emulsion composition as defined above, and subsequently removing the emulsion composition, for example by washing with water. In one embodiment, the emulsion composition is applied as a spray and is thickened prior to removal. This method leaves the skin cleansed but also smooth and moisturised.

The emulsion composition may be used as a medium for delivering compounds to the skin. For example, it may be used to restore the balance of lipids to skin which has become abnormal either as a result of environmental factors (such as exposure to detergents, ultraviolet irradiation or extremes of relative humidity) or through pathophysiological conditions such as eczema, ichthyosis and psoriasis.

Thus, there is also provided the use of an emulsion composition according to the present invention in the manufacture of a medicament for the treatment of such conditions.

The emulsion composition may be used as a vehicle for application of sunscreens, either as a sprayable lotion or as a cream. Use of an emulsion composition according to the present invention may give improved substantivity to skin and hair.

The emulsion may also be used to impregnate fibrous materials such as tissues to provide so-called 'baby-wipe' or 'wet-wipe' products. Tissues can be of various fibre types, either woven or non-woven and of rayon, vicose or cellulosic material.

Thus, there is also provided a fibrous material impregnated with an emulsion composition as defined above.

The properties of the emulsion compositions of the present invention may be modified to suit various types of cosmetic product.

For example, whilst normally naturally thin, the emulsion may be thickened by addition of, for example, a carbomer, montmorillonite clay, gum or alginate. The emulsion may be thickened to the point where it is no longer sprayable, and must instead be applied to the skin or hair as a cream or lotion. However, once applied, the rheological properties described above may still be obtained.

Alternatively, the naturally low viscosity of the emulsion may be retained whilst the rheology is modified to produce a less extreme shear-thickening on the skin or hair.

By altering the proportion of oil to water in the formulation whilst retaining the ratio of emulsifier to dimer acid, it is possible to alter the initial viscosity whilst maintaining the desired rheological properties on application to the skin or hair.

It has also surprisingly been found that dicarboxylic acids ('dimer acids') of formula I above are anti-free-radical agents, especially in respect of oxygen radicals.

Anti-free-radical agents are useful in cosmetics, pharmaceuticals and toiletries compositions of the present invention as they may help to reduce the build-up of undesirable free-radicals on the skin or hair.

This cosmetics, pharmaceuticals or toiletries composition of the present invention may or may not be in the form of an emulsion such as the shear-thickenable emulsions described above, and may or may not comprise an emulsifying agent. Suitably the composition is substantially free of spherical bilayer or multilayer membrane vesicles.

The term "cosmetics composition" as used herein includes, for example, skin creams, lotions, foundations, lipsticks and mascaras.

The term "toiletries composition" as used herein includes, for example, sunscreeen compositions, after sun products, cleansing lotions, soaps, shampoos, conditioners and deodorants/antiperspirants.

The term "pharmaceutical composition" as used herein includes, for example, ointments, creams, lotions, syrups and suspensions especially those for topical application.

The term "substantially free of spherical bilayer or multilayer membrane vesicles" as used herein denotes a composition comprising less than about 5%, suitably less than about 1%, preferably less than about 0.1%, especially less than about 0.01% by weight of said vesicles.

Particularly preferred cosmetics and toiletries compositions of the present invention are sunscreen compositions. Build-up of free-radicals is particularly likely when an individual is exposed to UV radiation, and use of a sunscreen composition comprising a dicarboxylic acid of formula I above may help to reduce such build-up.

The term "sunscreen composition" is used herein to include tanning lotions, sunscreens and sunblockers which are intended for topical application to provide protection against the sun's rays or other sources of untraviolet (UV) radiation.

Such sunscreen compositions may or may not comprise membrane vesicles, may or may not be in the form of emulsions as described above and may comprise one or more of a variety of sunscreening agents such as microfine titanium dioxide; microfine zinc oxide; para-aminobenzoic acids, esters and derivatives thereof (for example, 2-ethylhexyl para-dimethylaminobenzoate and the octyl ester of para-aminobenzoic acid); methoxy cinamate esters (such as 2-ethylhexyl para-methoxycinamate, 2-ethoxyethyl para-methoxycinamate or α,β-di-(paramethoxycinnamoyl)-α'-(2-ethylhexanoyl)-glycerin; benzophenones such as oxybenzone dibenzoylmethanes; and salicylate esters. Such sunscreening agents may typically be present in an amount of from 0.1% to 10% by weight of the composition.

The compositions of the present invention may be formulated in any of the known ways for such products e.g. as milks, lotions, creams, gels, sticks or aerosols. The preferred formulation types are water-in-oil or oil-in-water emulsions such as lotions or creams. The compositions may if desired be formulated as emulsions with shear-thickening properties as described above.

The water-in-oil and oil-in-water emulsions of the compositions of the present invention may comprise for example:
a) hydrocarbon oils such as paraffin or mineral oils;
b) waxes such as beeswax or paraffin wax;
c) natural oils such as sunflower oil, apricot kernel oil, shea butter or jojoba oil;
d) silicone oils such as dimethicone, cyclomethicone or cetyldimethicone;
e) fatty acid esters such as isopropyl palmitate or isopropyl myristate;
f) fatty alcohols such as cetyl alcohol or stearyl alcohol; or
g) mixtures thereof, for example, the blend of waxes available commercially under the Trade Name "Cutina" (Henkel).

In preferred water-in-oil compositions of the present invention the oil phase comprises 5 to 40%, more preferably 10 to 30% by weight of the composition. In preferred oil-in-water compositions of the present invention the oil phase comprises 5 to 30%, more preferably 10 to 20% by weight of the composition.

The emulsifiers used may be any emulsifiers known in the art for use in water-in-oil or oil-in-water emulsions. It has been found that particularly effective water-in-oil and oil-in-water compositions can be prepared by using an emulsifier or mixture of emulsifiers selected from known cosmetically acceptable emulsifiers which include:
a) sesquioleates such as sorbitan sesquioleate, available commercially for example under the Trade Name "Arlacel 83" (ICI), or polyglyceryl-2-sesquioleate;
b) ethoxylated esters of derivatives of natural oils such as the polyethoxylated ester of hydrogenated castor oil available commercially for example under the Trade Name "Arlacel 989" (ICI);
c) silicone emulsifiers such as silicone polyols available commercially for example under the Trade Name "ABIL WS08" (Th. Goldschmidt AG);
d) anionic emulsifiers such as fatty acid soaps e.g. potassium stearate and fatty acid sulphates e.g. sodium cetostearyl sulphate available commercially under the Trade Name "Dehydag" (Henkel);
e) ethoxylated fatty alcohols, for example the emulsifiers available commercially under the Trade Name "Brij" (ICI);
f) sorbitan esters, for example the emulsifiers available commercially under the Trade Name "Span" (ICI);
g) ethoxylated sorbitan esters, for example the emulsifiers available commercially under the Trade Name "Tween" (ICI) ;
h) ethoxylated fatty acid esters such as ethoxylated stearates, for example the emulsifiers available commercially under the Trade Name "Myrj" (ICI);
i) ethoxylated mono-, di-, and tri-glycerides, for example the emulsifiers available commercially under the Trade Name "Labrafil" (Alfa Chem.);
j) non-ionic self-emulsifying waxes, for example the wax available commercially under the Trade Name "Polawax" (Croda);
k) ethoxylated fatty acids, for example, the emulsifiers available commercially under the Trade Name "Tefose" (Alfa Chem.); or
l) mixtures thereof.

The amount of emulsifier present in the water-in-oil compositions of the present invention is preferably in the range 2 to 10%. Preferred water-in-oil emulsifiers are anionic or non-ionic emulsifiers. The amount of emulsifier present in the oil-in-water compositions of the present invention is preferably in the range 1 to 15%, more preferably 2 to 15%. Preferred oil-in-water emulsifiers include ethoxylated fatty acids and alcohols, ethoxylated stearates and ethoxylated triglycerides and mixtures thereof.

The compositions of the present invention may additionally comprise other components which will be well known to those skilled in the art. These include, for example, anti-perspirant actives such as aluminium salts, deodorant actives such as triclosan (available from Ciba-Geigy under the Trade Name "Irgasan") emollients such as isopropyl myristate or triglycerides of fatty acids e.g. lauric triglyceride or capric/caprylic triglyceride, such as the triglyceride available commercially from Huls UK under the Trade Name "Miglyol 810"; moisturisers such as D-panthenol; humectants such as glycerin or 1,3-butylene glycol; antioxidants such as DL-α-tocopherylacetate or butylated hydroxytoluene; emulsion stabilising salts such as sodium chloride, sodium citrate or magnesium sulphate; film formers to assist spreading on the surface of the skin such as alkylated polyvinylpyrrolidone e.g. available commercially from GAF under the Trade Name "Antaron"; thickeners such as acrylic acid polymers e.g. available commercially from B.F. Goodrich under the Trade Name "Carbopol" or modified celluloses e.g. hydroxyethylcellulose available commercially from Hercules under the Trade Name "Natrosol"; preservatives such as bronopol, sodium dehydroacetate, polyhexamethylenebiguanide hydrochloride, isothiazolone or diazolidinylurea; sequestering agents such as EDTA salts; perfumes and colourings.

It has also been found that the dimer acids defined in formula I above may help to improve substantivity of chemical sunscreening agents, especially water-soluble sunscreening agents, in the lower levels of the stratum corneum. Accordingly, in a further aspect of the present invention there is provided the use of a compound of formula I and a sunscreening agent in the manufacture of a sunscreen formulation to improve the substantivity of the sunscreening agent to the skin. Preferably the sunscreening agent is a water-soluble sunscreening agent.

The present invention is illustrated by the following non-limiting tests and examples (in which the abbreviation "POE" denotes "polyoxyethyl" and the abbreviation "PPG" denotes "polypropylene glycol"):

### Comparative Test A

Eight test formulations (I to VIII) were prepared as follows:

### Test formulation I

| Component | Concentration |
|---|---|
| POE (2) stearyl alcohol (available from ICI under Trade Name "Brij 72") | 2.5 |
| | |
| POE (21) stearyl alcohol (available from ICI under the Trade Name "Brij 721") | 1.5 |
| | |
| Dimer Acid (available from Unichema International under the Trade Name "Pripure 3780") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| Water | to 100 |

The formulation was adjusted to pH6 with sodium hydroxide.

### Test formulation II

| Component | Concentration (w/w%) |
|---|---|
| POE (2) stearyl (available from ICI under the Trade Name "Brij 72") | 2.5 |
| | |
| POE (21) steryl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/nanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| Octyl methoxy cinnamate (available from Givaudan-Roure under the Trade Name "Parsol MCX") | 4 |
| | |
| Water | to 100 |

The formulation was adjusted to pH 6 with sodium hydroxide.

### Test formulation III

| Component | Concentration (w/w%) |
|---|---|
| POE (2) stearyl alcohol (available from ICI under the Trade Name "Brij 72") | 2.5 |
| | |
| POE (21) stearyl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| 2-phenyl benzimidazole-5-sulphonic acid (available from Givaudan-Roure under the Trade Name "Parsol HS") | 4 |
| | |
| Water | to 100 |

The formulation was adjusted to pH 6 with sodium hydroxide.

### Test formulation IV

| Component | Concentration (w/w%) |
|---|---|
| POE (2) stearyl alcohol (available from ICI under the Trade Name "Brij 72") | 2.5 |
| | |
| POE stearyl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| Birch extract (available under the Trade Name "HP Herbasol Betula") | 0.3 |
| | |
| Water | to 100 |

The formulation was adjusted to pH 6 with sodium hydroxide.

### Test formulation V

| Component | Concentration (w/w%) |
|---|---|
| POE (2) stearyl alcohol (available from ICI under the Trade Name "Brij 72") | 2.5 |
| | |
| POE (21) stearyl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| Water | to 100 |

The formulation was adjusted to pH 6 with sodium hydroxide.

### Test formulation VI

| Component | Concentration (w/w%) |
|---|---|
| POE (2) stearyl alcohol (available from ICI under the Trade Name "Brij 72") | 2.5 |
| | |
| POE (21) stearyl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| Dimer Acid (available from Unichema International under the Trade Name "Pripure 3780" | 1.5 |
| | |
| Birch extract (available under the Trade Name "HP Herbasal Betula") | 0.3 |
| | |
| Water | to 100 |

The formulation was adjusted to pH 6 with sodium hydroxide.

### Test formulation VII

| Component | Concentration (w/w%) |
|---|---|
| POE (2) stearyl alcohol (available from ICI under the Trade Name "Brij 72") | 2.5 |
| | |
| POE (21) stearyl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| Octyl methoxy cinnamate (available from Givaudan-Roure under the Trade Name "Parsol MCX") | 4.0 |
| | |
| Dimer Acid (available from Unichema International under the Trade Name "Pripure 3780" | 1.5 |
| | |
| Water | to 100 |

The formulation was adjusted to pH 6 with sodium hydroxide.

### Test formulation VIII

| Component | Concentration (w/w%) |
|---|---|
| POE (2) stearyl alcohol (available from ICI under the Trade Name "Brij 72") | 2.5 |
| | |
| POE (21) stearyl alcohol (available from ICI under the Trade Name "Brij 721") | 1.5 |
| | |
| 2-hexyl decyl oleate (available from Condea under the Trade Name "Isofol-16-oleate") | 2.5 |
| | |
| 2-octyl dodecyl oleate (available from Condea under the Trade Name "Isofol-20-oleate") | 1.5 |
| | |
| Mixture of C₁₀₋₃₀ cholesterol/lanosterol esters (available from Croda Chemical under the Trade Name "Super Sterol Ester") | 1.0 |
| | |
| Mixture of squalane and unsaponifiables (available from Unichema International under the Trade Name "Prisorine 3759") | 2.0 |
| | |
| Propyl paraben | 0.1 |
| | |
| Methyl paraben | 0.1 |
| | |
| 2-phenyl-benzimidozole-5-sulphonic acid (available from Givaudan-Roure under the Trade Name "Parsol HS") | 4 |
| | |
| Dimer Acid (available from Unichema International under the Trade Name "Pripure" | 1.5 |
| | |
| Sodium hydroxide | 0.56 |
| | |
| Water | to 100 |

These formulations were tested for resistance to UV-induced free radical activity as follows;

Two 20 g aliquots were taken from each formulation and each placed in separate UV-transparent plastic containers. One aliquot of each formulation was exposed to radiation from a solar simulator at a level of about 5.6 mW/cm² for a period of about one hour. The other aliquot was left to stand unirradiated for the same period.

When irradiation was complete, the oxygen tension of each aliquot of each formulation was measured with a calibrated oxygen electrode (model 97-08-99 from Orion). Each aliquot was tested three times at intervals of about one minute. The oxygen electrode was thoroughly cleaned with distilled water between measurements. Results are set out in Table 1.

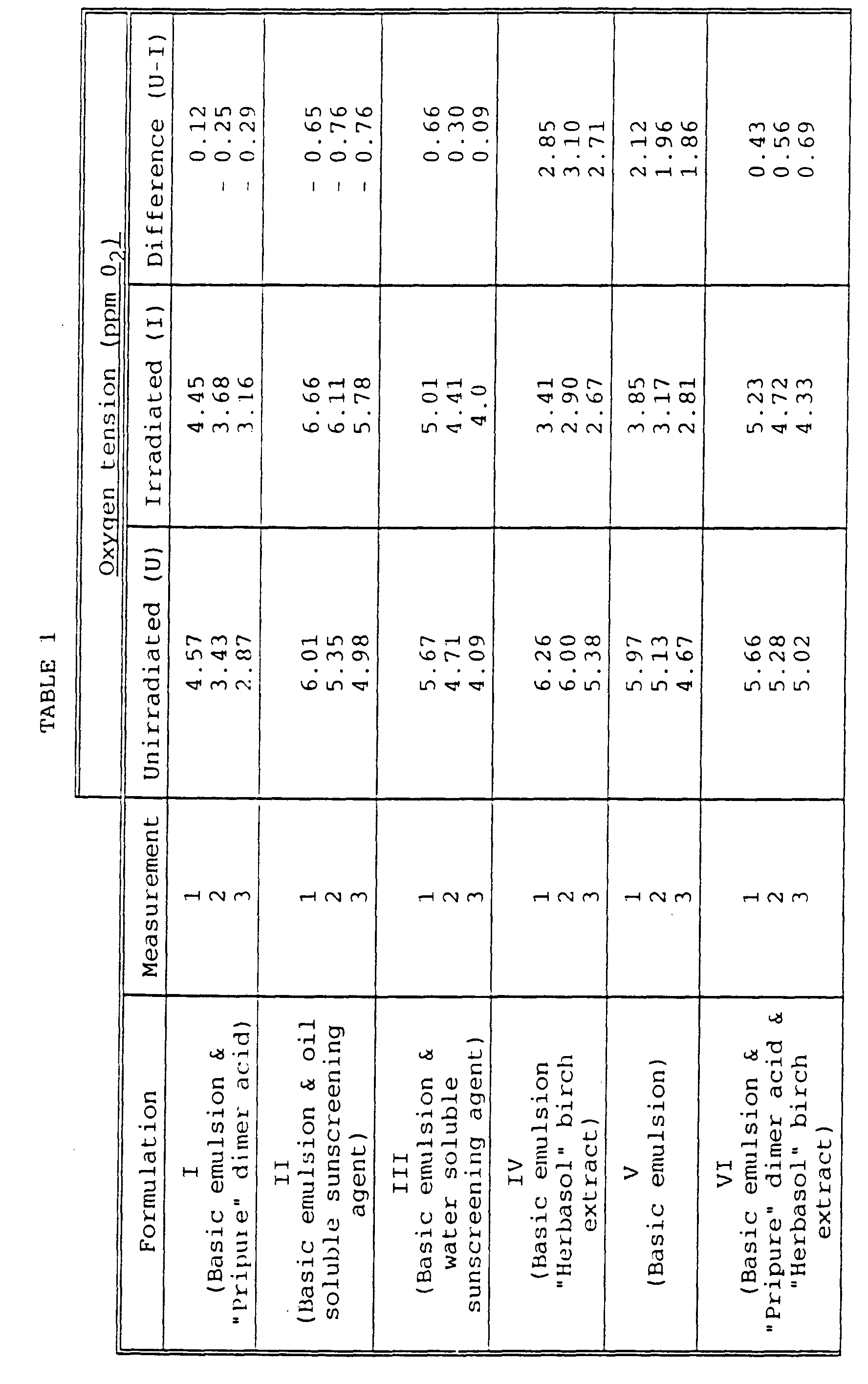

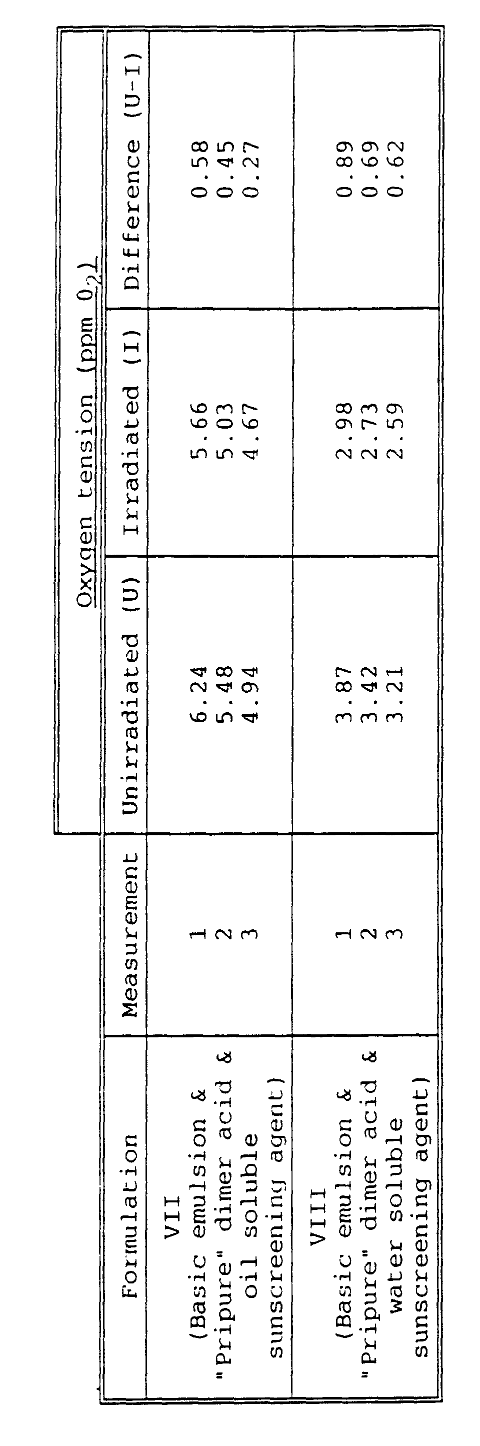

Reductions in oxygen tension following UV irradiation are interpreted as oxidation of unsaturated fatty acids in the emulsion as a result of free radical activity. Formulations I, VII and VIII (containing "Pripure" dimer acid) are thus seen to have an apparent anti-free-radical effect which may have a "scavenging" mechanism. Such an effect is also seen in formulations II, III, VII and VIII (containing sunscreens) but this may be due to the absorbance of UV radiation by the sunscreen component rather than by free radical scavenging action.

A further set of oxygen tension measurements was taken after a further period of about 2 hours, except that only one measurement was taken for each aliquot. Results are set out in Table 2.

It will be noted that the results generally follow the trends shown in Table 1 above, except that in Table 2 the known free radical scavenger, birch extract, is seen to have an apparent anti-free-radical effect.

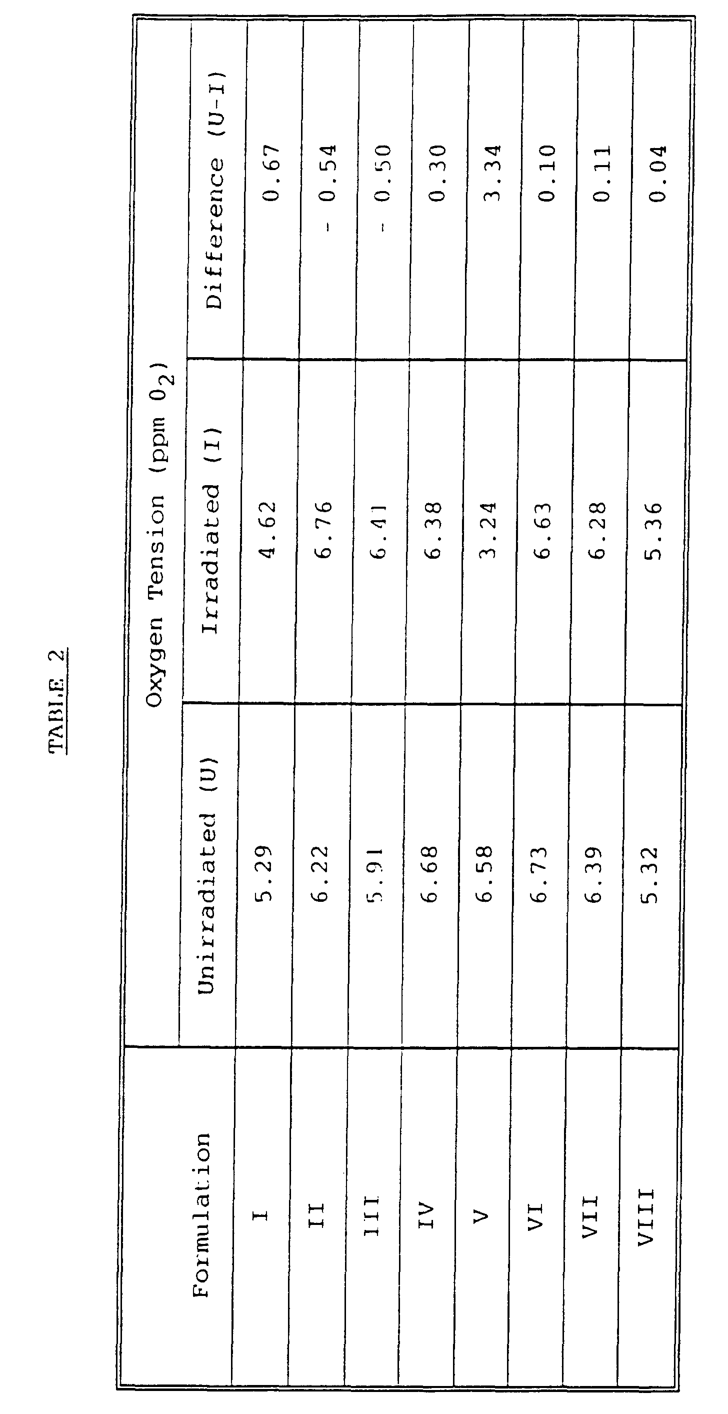

### Comparative Test B

Formulations II, III, VII and VIII from Comparative Test A above were tested as follows:

In each case 40 µl of the formulation was applied to a separate 60 x 60 mm skin surface on the forearm of a volunteer. The formulation was spread over the skin surface using a glass rod and allowed to dry. Each surface was divided into four quarters (15 mm x 15 mm; designated A, B, C and D respectively.

Three successive strips of skin were then removed from each of the quarters A and B by applying and then removing three circles of adhesive tape (available under the Trade Name "D-Squame") in turn from the same area of skin. After removal each portion of tape was mixed vigorously with 3 ml denatured ethanol in a small conical flask for 1 minute and the optical density (at 307 nm) of the resulting ethanolic solution was determined using a UV-visible spectrophotometer.

Each skin surface was then washed with warm water (10 x 500 ml) which was run down the forearm, over the surfaces. The treated skin was allowed to dry without rubbing and then mopped gently with a single application of a paper towel. The above stripping process was then carried out on quarters C and D.

In each case optical density (307 nm) reading in respect of the first circle of D-Squame" tape removed is proportional to the amount of sunscreen retained at level 1 of the stratum corneum, the second circle to the amount retained at level 2 and the third circle to the amount retained at level 3. Results are set out in Table 3.

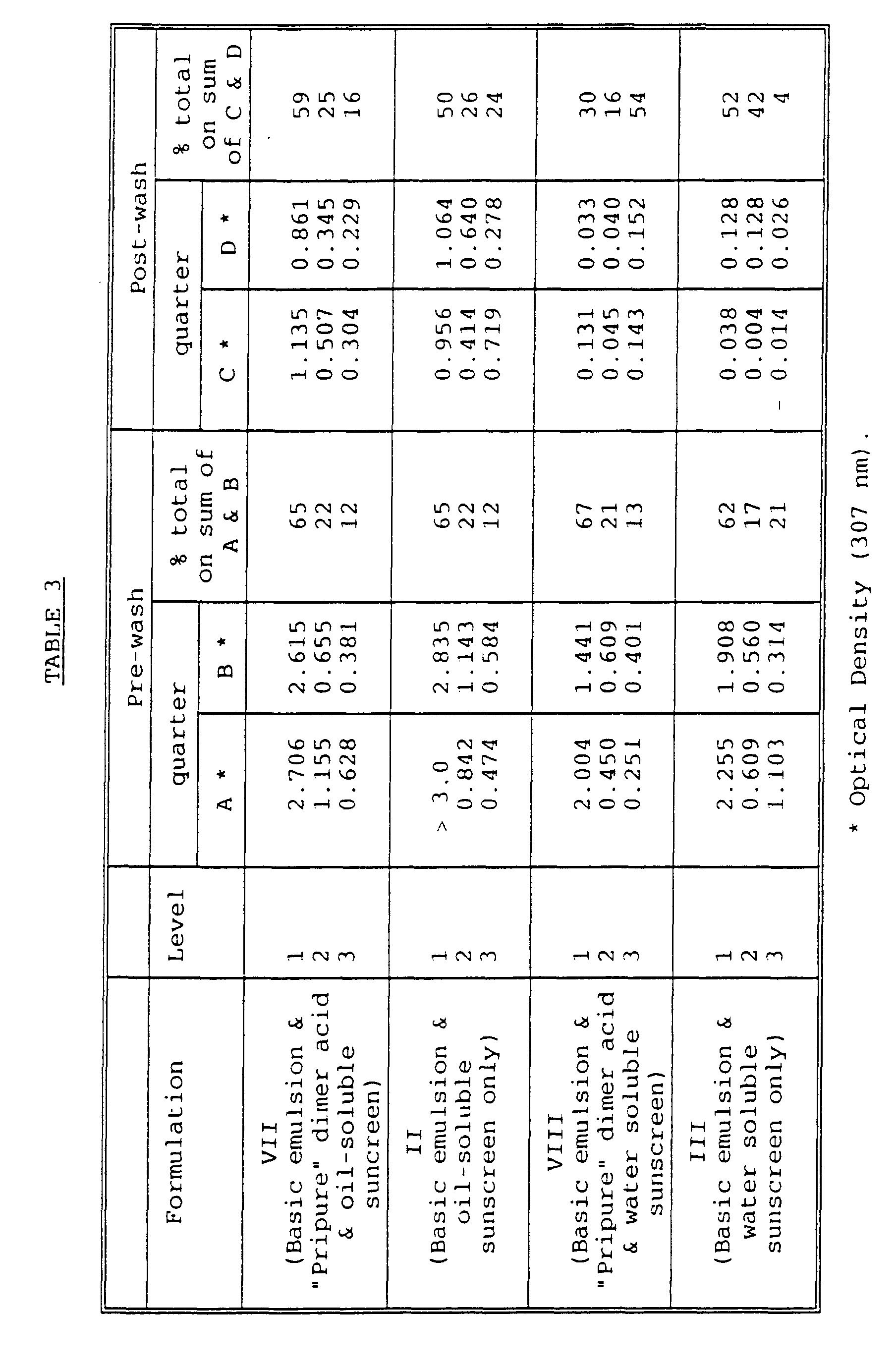

These results suggest that the "Pripure" dimer acid increases the proportion of water soluble sunscreening agent which is retained at lower levels of the stratum corneum.

### Example 1 - Pump Spray delivered emulsion

| Oil Phase | w/w % |
|---|---|
| POE (2) stearyl alcohol | 2.0% |
| | |
| POE (21) stearyl alcohol | 1.0% |
| | |
| Dimer acid (available from Unichema International International under the Trade Name "Pripure 3780") | 1.0% |
| | |
| Cholesterol | 0.15% |
| | |
| Ceramide (available from Sederma, France under the Trade Designation "HO3") | 0.10% |

| Aqueous chase | |
|---|---|
| Aqueous sodium hydroxide/potassium hydroxide solution (2.5% : 2.5%) | to pH 6.5 |
| | |
| Water | to 100% |

### Method

The oil phase components were melted together at 80°C and thoroughly mixed to produce clear dark yellow fluid. Water at approx 77°C was agitated using a Silverson mixer and the oil phase components were added at equivalent temperature. Mixing was continued at this temperature for 15 minutes. The emulsion was then stir cooled, pH adjusted to approximately 6.5 using the alkali solution, and made up to 100% with water.

This product was thin enough to be pumped by an aerosol onto the skin prior to rubbing in and thickening into a cream with a dry, non-tacky, non-greasy feel.

### Comparative Example A

Emulsions were formed as described under Example 1 above except that the ethoxylated stearyl alcohol was replaced variously by ethoxylated lauryl alcohol, ethoxylated stearic acid or ethoxylated sorbitan monostearate. The resulting emulsions failed to show the rheological properties of the emulsion of Example 1.

### Example 2 - Wash-off Cleanser

| Oil phase | w/w % |
|---|---|
| POE (2) stearyl alcohol | 5.0% |
| | |
| POE (21) stearyl alcohol | 2.5% |
| | |
| Dimer acid (available from Unichema International under the Trade Name "Pripure 3780") | 2.5% |
| | |
| Cholesterol | 0.375% |
| | |
| Ceramide (available from Sederma under the Trade Designation "HO3") | 0.25% |

| Aqueous phase | |
|---|---|
| Aqueous sodium hydroxide/potassium hydroxide solution (2.5% : 2.5%) | to pH 6 |
| | |
| Water | to 100% |

### Method

The product was formulated substantially as described in Example 1. pH was adjusted to 6 using the alkali solution prior to making up to weight. The milky product, once rubbed into the skin, leaves a dry finish which when rubbed under running water gives a slightly soapy feel. The skin is cleansed but left smooth and moisturised after use.

### Example 3 - Mixed 'skin lipid' emulsion

| Oil phase | w/w% |
|---|---|
| POE (2) stearyl alcohol | 2.0% |
| | |
| POE (21) stearyl alcohol | 1.0% |
| | |
| Dimer acid (available from Unichema International under the Trade "Name Pripure 3780") | 1.14% |
| | |
| Dimer acid ester (available from Unichema International under the Trade Name "Pripure DIPD 3786") | 0.5% |
| | |
| Ceramide (available from Sederma under the Trade Designation "HO3") | 0.1% |
| | |
| Cholesterol | 0.15% |
| | |
| Sterol ester | 0.15% |
| | |
| Evening primrose oil | 0.2% |

| Aqueous phase | w/w% |
|---|---|
| Aqueous sodium hydroxide/potassium hydroxide solution (2.5% : 2.5%) | to pH 6 |
| | |
| Water | to 100% |

The product was formulated substantially as described in Example 1. pH was adjusted to 6 using the alkali solution prior to making up to weight.

### Example 4 - Sunscreen emulsion

| Oil phase | w/w% |
|---|---|
| POE (2) stearyl alcohol | 2.0% |
| | |
| POE (21) stearyl alcohol | 1.0% |
| | |
| Dimer acid (available from Unichema International under the Trade Name "Pripure 3780") | 1.0% |
| | |
| Coated microfine titanium dioxide (available from Tayca under the Trade Name "MT100T") | 1.0% |

| Aqueous phase | w/w% |
|---|---|
| Acrylates t-octyl propanamide copolymer (available from National Starch under the Trade Name "Dermacryl 79") | 0.1% |
| | |
| Aqueous sodium hydroxide solution (1M) | to pH 6 |
| | |
| Water | to 100% |

### Method

The acrylates copolymer was suspended in water and subjected to vigorous agitation for 20 minutes. The water and oil phases were separately heated to approximately 75°C and mixed under high shear for 15 minutes. The emulsion was stir cooled, adjusted to pH 6 using the alkali solution and made up to 100% with water.

### Example 5 - Baby lotion wipes

| Oil phase | w/w % |
|---|---|
| POE (2) stearyl alcohol | 2.04% |
| | |
| POE (21) stearyl alcohol | 1.03% |
| | |
| Dimer acid (available from Unichema International under the Trade Name "Pripure 3780") | 1.06% |
| | |
| Cholesterol USP | 0.25% |
| | |
| Heptamethyl nonane (available from ICI under the Trade Name "Arlamol HD") | 0.75% |
| | |
| Ceramide (available from Sederma under the Trade Designation "HO3") | 0.05% |

| Aqueous phase | w/w % |
|---|---|
| Allantoin BPC '34 | 0.05% |
| | |
| Cetrimonium bromide | 0.3 % |
| | |
| Polyhexamethylenediguanide hydrochloride (available from ICI under the Trade Name "Arlagard E") | 0.3 % |
| | |
| Aqueous sodium hydroxide solution (1M) | to pH 6 |
| | |
| Water | to 100% |

### Method

The oil phase was made up and mixed with water substantially as described in Example 1. Separate aqueous solutions of Allantoin and Cetrimonium bromide were added with stirring. To the resulting mixture polyhexamethylenediguanide hydrochloride was added as an aqueous solution to the required concentration. The resulting emulsion was stir-cooled, pH adjusted to approximately 6.0 using the alkali solution, and made up to 100% with water. The emulsion permeated freely through a roll of tissues to provide a 'wet-wipe'/'baby-wipe' product.

### Example 6 - Cream cleanser

| Oil phase | w/w % |
|---|---|
| POE (2) stearyl alcohol | 5.0% |
| | |
| POE (21) stearyl alcohol | 2.5% |
| | |
| Dimer acid (available from Unichema International under the Trade Name "Pripure 3780") | 2.5% |
| | |
| Avocado unsaponifiables (available from Croda Chemical under the Trade Name "Avocadin") | 0.4% |
| | |
| Ceramide (vegetable source) available from Bioetica, France | 0.1 % |
| | |
| Silicone fluid (20 centistokes) | 0.2 % |

| Aqueous phase | w/w% |
|---|---|
| Carboxypolymethylene (available from BF Goodrich under the Trade Name "Carbopol 934") | 0.2% |
| | |
| Sodium ethylenediaminetetraacetate | 0.04% |
| | |
| Aqueous sodium hydroxide solution (1M) | to pH 6 |
| | |
| Water | to 100% |

The carboxypolymethylene and sodium ethylenediamine tetraacetate were predissolved using Silverson mixing (30 minutes) in approximately 80% of the final volume of water, to provide an aqueous phase. The oil phase was made up and mixed with the aqueous phase substantially as described in Example 1. The resulting emulsion was stir-cooled, pH adjusted to approximately 6 using the alkali solution, and made up to 100% with water. The emulsion was found to be thicker than the product of Example 1, but still showed the same rheological properties when applied to the skin.

### Example 7 - Cleansing lotion

| Oil phase | w/w% |
|---|---|
| POE (2) stearyl alcohol | 2.0% |
| | |
| POE (21) stearyl alcohol | 1.0% |
| | |
| Dimer acid (available from Unichema International under the Trade Name "Pripure 3780") | 1.1% |
| | |
| Cholesterol USP | 0.15% |
| | |
| Ceramide (available from Sederma under the Trade Designation "HO3") | 0.04% |
| | |
| Light liquid paraffin | 0.20% |
| | |
| Heptamethyl nonane (available from ICI under the Trade Name "Arlamol HD") | 0.40% |

| Aqueous phase | w/w % |
|---|---|
| Aqueous sodium hydroxide solution (1M) | to pH 6.0 |
| | |
| Water | to 100% |

The product was formulated substantially as described in Example 1. The finished product was sprayable and when rubbed into the skin it exhibited some thickening but did not impart the heavy 'drag' sometimes seen with Example 1 above. The final skin feel before and after washing was almost identical to that obtained by use of the formulation of Example 1.

### Example 8 - Barrier cream

| Oil phase | w/w % |
|---|---|
| POE (2) stearyl alcohol | 12% |
| | |
| POE (21) stearyl alcohol | 5% |
| | |
| Dimer Acid (available from Unichema International under the Trade Name "Pripure 3780") | 5% |
| | |
| Cholesterol USP | 1% |
| | |
| Oleopolymeric emollient (available from Croda Chemical under the Trade Name "Supermol S") | 1.5% |
| | |
| Beeswax substitute | 0.75% |
| | |
| Silicone fluid (300 centistokes) | 2% |
| | |
| Arachidyl propionate (available from Nobel under the Trade Name "Waxenol 801") | 1.5% |
| | |
| Ceramide (vegetable origin) available from Sederma | 0.75% |
| | |
| Heptamethyl nonane (available from ICI under the Trade Name "Arlamol HD") | 0.5% |

| Aqueous phase | w/w% |
|---|---|
| Aqueous sodium hydroxide solution (1M) | to pH 6.0 |
| | |
| Water | to 100% |

The product was formulated substantially as described in Example 1. The finished product was too thick to be sprayable but still exhibited the rheological properties described above in respect of Example 1 and formed a highly protective layer on the skin.

### Example 9 - Anti-perspirant emulsion

| Oil Phase | w/w% |
|---|---|
| POE (2) stearyl alcohol | 2 |
| | |
| POE (21) stearyl alcohol | 1 |
| | |
| Dimer Acid (available from Unichema International under the Trade Name "Pripure 3780") | 1 |
| | |
| Di-isopropyl adipate | 3 |
| | |
| PPG (15) stearyl ether (available from ICI under the Trade Name "Arlamol E") | 3 |
| | |
| Cyclomethicone (available from Dow Corning under the Trade Designation "DC345") | 5 |
| | |
| Aluminium chlorhydrate (available from Reheis, Ireland under the Trade Name "Micro-Dry") | 5 |

| Aqueous Phase | |
|---|---|
| Water | to 100% |

### Method

The oils were heated to approximately 75°C to produce a clear liquid. The cyclomethicone was then added and the resulting mixture subjected to high shear mixing. The aluminium chlorhydrate was then added with continued high shear mixing.

The water was heated to approximately 75°C and to this were added the oils (re-heated to 75°C) with continued high shear mixing for about 10 minutes.

The resulting mixture was cooled rapidly (by so-called "crash" cooling) with continued mixing to provide the desired product.

The emulsion obtained comprises a stable white milky fluid with suspended, oil-bound aluminium particles. Microscopic examination showed these to be within liquid crystalline arrays. Delivery of this emulsion from a roll-on provides a quick-drying anti-perspirant with good skin feel properties.

Dispensing the emulsion via a "roller" mechanism gives a shearing action so that the product is delivered in a ready-thickened state on the skin.

## Claims

1. A cosmetics, pharmaceutical or toiletries composition comprising a linear dicarboxylic acid of the formula I 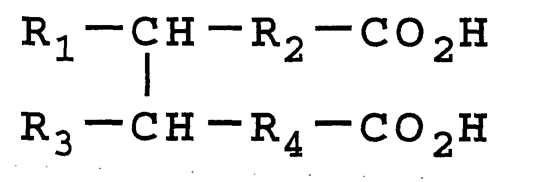 or an acceptable salt thereof;
wherein R₁ and R₃ are each independently C₆ to C₁₀ alkyl and R₂ and R₄ are each independently C₆ to C₁₀ alkylene, with the proviso that the acid comprises one or more unsaturated carbon-carbon bonds and is substantially free of other polymeric acids.

2. A composition as claimed in claim 1 wherein R₁, R₂, R₃ and R₄ all have the same chain length.

3. A composition as claimed in claim 2 wherein the dicarboxylic acid is dilinoleic acid.

4. A composition as claimed in any one of the preceding claims wherein the dicarboxylic acid is present in an amount of from about 0.1% to about 30% by weight of the composition.

5. A composition as claimed in claim 4 wherein the dicarboxylic acid is present in an amount of from about 0.2% to about 10% by weight of the composition.

6. A composition as claimed in any one of the preceding claims in the form of a sunscreen formulation comprising a sunscreening agent.

7. A composition as claimed in Claim 6 wherein the sunscreening agent is present in an amount of from about 0.1% to about 10% by weight of the composition.

8. A composition as claimed in any one of the preceding claims which is substantially free of spherical bilayer or multilayer membrane vesicles.

9. A composition as claimed in any one of the preceding claims in the form of an emulsion.

10. A composition as claimed in Claim 9 wherein the emulsion contains an emulsifying agent which is an alkoxylated emulsifier.

11. A composition as claimed in Claim 10 wherein the emulsifying agent is an alkoxylated C₁₄ to C₂₂ alkanol.

12. A composition as claimed in any one of claims 9 to 11 wherein the emulsifying agent is a mixture of alkoxylated C₁₄ to C₂₂ alkanols having different hydrophilic/lipophilic balances (HLBs).

13. A composition as claimed in Claim 12 wherein the emulsifying agent is a mixture of a polyethoxylated C₁₄ to C₂₂ alkanol bearing a chain of 2 to 10 ethoxy groups (HLB between about 4 and about 12) and a polyethoxylated C₁₄ to C₂₂ alkanol bearing a chain of 10 to 40 ethoxy groups (HLB between about 10 and about 20).

14. A composition as claimed in Claim 13 wherein the emulsifying agent is a mixture of POE (2) stearyl alcohol and POE (21) stearyl alcohol in a ratio of about 2:1.

15. A composition as claimed in any one of Claims 10 to 14 wherein the emulsifying agent is present in an amount of from about 0.5% to about 20% by weight of the emulsion.

16. A composition as claimed in any one of Claims 10 to 15 wherein the emulsifying agent and the dicarboxylic acid are present in a weight ratio of about 5:1 to about 1:1.

17. An emulsion as claimed in any one of Claims 10 to 16 wherein the emulsion is an oil-in-water emulsion.

18. A roller dispenser comprising a composition as claimed in any of claims 10 to 17.

19. An aerolising means comprising a composition as claimed in any one of claims 10 to 17.

20. A fibrous material impregnated with a composition as claimed in any one of claims 10 to 17.

21. Use of a an emulsion comprising a linear dicarboxylic acid of formula I as claimed in any one of claims 1 to 3, in the manufacture of a medicament for the restoration of the balance of lipids to skin which has become abnormal either as a result of environmental factors or through pathophysiological conditions.

22. Use of a dicarboxylic acid of fomula I represented in any one of claims 1 to 3, in the manufacture of a composition to reduce the build-up of undesirable free radicals on the skin and hair.

23. Use of a dicarboxylic acid of formula I represented in any one of claims 1 to 3 and a sunscreeening agent in the manufacture of a sunscreen formulation to improve the substantivity of the sunscreening agent to the skin.

## Patentansprüche

1. Kosmetische, pharmazeutische oder Hygieneartikel-Zubereitung, umfassend eine lineare Dicarbonsäure der Formel I 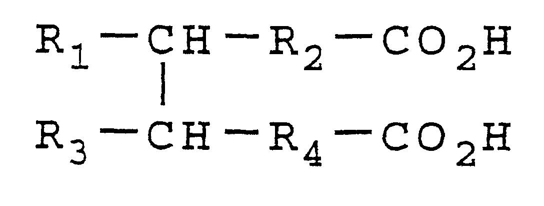 oder ein annehmbares Salz derselben;
worin R₁ und R₃ jeweils unabhängig C₆-C₁₀-Alkyl sind und R₂ und R₄ jeweils unabhängig C₆-C₁₀-Alkylen darstellen, mit der Maßgabe, dass die Säure eine oder mehrere ungesättigte KohlenstoffKohlenstoff-Bindungen umfasst und im Wesentlichen frei von anderen polymeren Säuren ist.

2. Zubereitung gemäß Anspruch 1, worin R₁, R₂, R₃ und R₄ alle dieselbe Kettenlänge haben.

3. Zubereitung gemäß Anspruch 2, worin die Dicarbonsäure Dilinolensäure ist.

4. Zubereitung gemäß einem der vorstehenden Ansprüche, worin die Dicarbonsäure in einer Menge von etwa 0,1 Gew.-% bis etwa 30 Gew.-% der Zubereitung vorhanden ist.

5. Zubereitung gemäß Anspruch 4, worin die Dicarbonsäure in einer Menge von etwa 0,2 Gew.-% bis etwa 10 Gew.-% der Zubereitung vorhanden ist.

6. Zubereitung gemäß einem der vorstehenden Ansprüche in Form einer Sonnenschutzformulierung, umfassend ein Sonnenschutzmittel.

7. Zubereitung gemäß Anspruch 6, worin das Sonnenschutzmittel in einer Menge von etwa 0,1 Gew.-% bis etwa 10 Gew.-% der Zubereitung vorhanden ist.

8. Zubereitung gemäß einem der vorstehenden Ansprüche, die im Wesentlichen frei von kugelförmigen zweischichtigen oder mehrschichtigen Membranvesikeln ist.

9. Zubereitung gemäß einem der vorstehenden Ansprüche in Form einer Emulsion.

10. Zubereitung gemäß Anspruch 9, worin die Emulsion ein emulgierendes Mittel enthält, bei dem es sich um einen alkoxylierten Emulgator handelt.

11. Zubereitung gemäß Anspruch 10, worin das emulgierende Mittel ein alkoxyliertes C₁₄- bis C₂₂-Alkanol ist.

12. Zubereitung gemäß einem der Ansprüche 9 bis 11, worin das emulgierende Mittel eine Mischung von alkoxylierten C₁₄- bis C₂₂-Alkanolen mit verschiedenen Hydrophil/Lipophil-Gleichgewichten (HLBs = Hydrophilic/Lipophilic Balances) ist.

13. Zubereitung gemäß Anspruch 12, worin das emulgierende Mittel eine Mischung eines polyethoxylierten C₁₄- bis C₂₂-Alkanols mit einer Kette von 2 bis 10 Ethoxygruppen (HLB zwischen etwa 4 und etwa 12) und einem polyethoxylierten C₁₄- bis C₂₂-Alkanol mit einer Kette von 10 bis 40 Ethoxygruppen (HLB zwischen etwa 10 und etwa 20) ist.

14. Zubereitung gemäß Anspruch 13, worin das emulgierende Mittel eine Mischung eines polyethoxylierten (2) Stearylalkohols und eines polyethoxylierten (21) Stearylalkohols in einem Verhältnis von etwa 2:1 ist.

15. Zubereitung gemäß einem der Ansprüche 10 bis 14, worin das emulgierende Mittel in einer Menge von etwa 0,5 Gew.-% bis etwa 20 Gew.-% der Emulsion vorhanden ist.

16. Zubereitung gemäß einem der Ansprüche 10 bis 15, worin das emulgierende Mittel und die Dicarbonsäure in einem Gewichtsverhältnis von etwa 5:1 bis etwa 1:1 vorhanden sind.

17. Emulsion gemäß einem der Ansprüche 10 bis 16, worin die Emulsion eine Öl-in-Wasser-Emulsion darstellt.

18. Rolldispenser, umfassend eine Zubereitung gemäß einem der Ansprüche 10 bis 17.

19. Vorrichtung zur Aerosolbildung, umfassend eine Zubereitung gemäß einem der Ansprüche 10 bis 17.

20. Faseriges Material, imprägniert mit einer Zubereitung gemäß einem der Ansprüche 10 bis 17.

21. Verwendung einer Emulsion, umfassend eine lineare Dicarbonsäure der Formel I, wie in einem der Ansprüche 1 bis 3 beansprucht, bei der Herstellung eines Medikaments für die Wiederherstellung des Lipidgleichgewichts der Haut, das entweder als Folge von Umweltfaktoren oder über pathophysiologische Bedingungen anormal geworden ist.

22. Verwendung einer Dicarbonsäure der Formel I, dargestellt in einem der Ansprüche 1 bis 3, bei der Herstellung einer Zusammensetzung zur Verringerung des Aufbaus unerwünschter freier Radikaler auf der Haut und dem Haar.

23. Verwendung einer Dicarbonsäure der Formel I, dargestellt in einem der Ansprüche 1 bis 3, und eines Sonnenschutzmittels bei der Herstellung einer Sonnenschutzformulierung zur Verbesserung der Substantivität des Sonnenschutzmittels gegenüber der Haut.

## Revendications

1. Composition cosmétique, composition pharmaceutique ou composition de toilette, comprenant un acide dicarboxylique linéaire de formule I 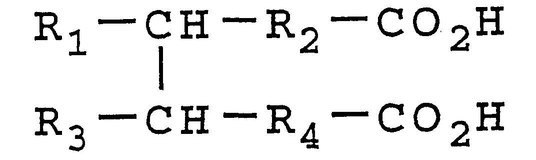 ou un de ses sels acceptables ;
formule dans laquelle R₁ et R₃ représentent chacun, indépendamment, un groupe alkyle en C₆ à C₁₀ et R₂ et R₄ représentent chacun, indépendamment, un groupe alkylène en C₆ à C₁₀, sous réserve que l'acide comprenne une ou plusieurs liaisons carbone-carbone insaturées et soit pratiquement dépourvu d'autres acides polymères.

2. Composition suivant la revendication 1, dans laquelle R₁, R₂, R₃ et R₄ ont tous la même longueur de chaîne.

3. Composition suivant la revendication 2, dans laquelle l'acide dicarboxylique est l'acide dilinoléique.

4. Composition suivant l'une quelconque des revendications précédentes, dans laquelle l'acide dicarboxylique est présent en une quantité d'environ 0,1 % à environ 30 % en poids de la composition.

5. Composition suivant la revendication 4, dans laquelle l'acide dicarboxylique est présent en une quantité d'environ 0,2 % à environ 10 % en poids de la composition.

6. Composition suivant l'une quelconque des revendications précédentes, sous forme d'une formulation d'écran solaire comprenant un agent écran solaire.

7. Composition suivant la revendication 6, dans laquelle l'agent écran solaire est présent en une quantité d'environ 0,1 % à environ 10 % en poids de la composition.

8. Composition suivant l'une quelconque des revendications précédentes, qui est pratiquement dépourvue de vésicules formées de membranes sphériques en deux couches ou plus de deux couches.

9. Composition suivant l'une quelconque des revendications précédentes, sous forme d'une émulsion.

10. Composition suivant la revendication 9, dans laquelle l'émulsion contient un agent émulsionnant qui est un agent émulsionnant alkoxylé.

11. Composition suivant la revendication 10, dans laquelle l'agent émulsionnant est un alcanol en C₁₄ à C₂₂ alkoxylé.

12. Composition suivant l'une quelconque des revendications 9 à 11, dans laquelle l'agent émulsionnant est un mélange d'alcanols en C₁₄ à C₂₂ alkoxylés ayant deux valeurs différentes d'équilibre hydrophile-lipophile (EHL).

13. Composition suivant la revendication 12, dans laquelle l'agent émulsionnant est un mélange d'un alcanol en C₁₄ à C₂₂ polyéthoxylé portant une chaîne de 2 à 10 groupes éthoxy (EHL d'environ 4 à environ 12) et d'un alcanol en C₁₄ à C₂₂ polyéthoxylé portant une chaîne de 10 à 40 groupes éthoxy (EHL d'environ 10 à environ 20).

14. Composition suivant la revendication 13, dans laquelle l'agent émulsionnant est un mélange d'alcools POE (2)stéaryliques et d'alcools POE(21)stéaryliques en un rapport d'environ 2:1.

15. Composition suivant l'une quelconque des revendications 10 à 14, dans laquelle l'agent émulsionnant est présent en une quantité d'environ 0,5 % à environ 20 % en poids de l'émulsion.

16. Composition suivant l'une quelconque des revendications 10 à 15, dans laquelle l'agent émulsionnant et l'acide dicarboxylique sont présents en un rapport pondéral compris dans l'intervalle d'environ 5:1 à environ 1:1.

17. Emulsion suivant l'une quelconque des revendications 10 à 16, qui est une émulsion huile-dans-eau.

18. Distributeur à rouleau comprenant une composition suivant l'une quelconque des revendications 10 à 17.

19. Moyen de production d'aérosol comprenant une composition suivant l'une quelconque des revendications 10 à 17.

20. Matière fibreuse imprégnée d'une composition suivant l'une quelconque des revendications 10 à 17.

21. Utilisation d'une émulsion comprenant un acide dicarboxylique linéaire de formule I suivant l'une quelconque des revendications 1 à 3, dans la production d'un médicament destiné au rétablissement de l'équilibre lipidique de la peau qui est devenu anormal en résultat de facteurs ambiants ou d'états physiopathologiques.

22. Utilisation d'un acide dicarboxylique de formule I représenté dans l'une quelconque des revendications 1 à 3, dans la production d'une composition destinée à réduire l'accumulation de radicaux libres indésirables sur la peau et les cheveux.

23. Utilisation d'un acide dicarboxylique de formule I représenté dans l'une quelconque des revendications 1 à 3 et d'un agent écran solaire dans la production d'une formulation d'écran solaire pour améliorer la substantivité de l'agent écran solaire vis-à-vis de la peau.
